Europäisches Patentamt

European Patent Office

Office européen des brevets

(19)

(11) Veröffentlichungsnummer: **0 341 470**

**A1**

(12) ## EUROPÄISCHE PATENTANMELDUNG

(21) Anmeldenummer: 89107298.5

(22) Anmeldetag: 22.04.89

(51) Int. Cl.⁴: **G01N 33/58 , G01N 33/68 , G01N 33/531 , G01N 33/96**

(30) Priorität: 03.05.88 DE 3814892

(43) Veröffentlichungstag der Anmeldung:
15.11.89 Patentblatt 89/46

(84) Benannte Vertragsstaaten:
AT BE CH DE ES FR GB GR IT LI LU NL SE

(71) Anmelder: **VIROTECH SYSTEM-DIAGNOSTIKA GmbH**
**Löwenplatz 5**
**D-6090 Rüsselsheim(DE)**

(72) Erfinder: **Der Erfinder hat auf seine Nennung verzichtet**

(74) Vertreter: **Patentanwälte RUFF, BEIER und SCHÖNDORF**
**Neckarstrasse 50**
**D-7000 Stuttgart 1(DE)**

(54) Kit und Verfahren zur quantitativen Bestimmung von Antikörpern.

(57) Kit und ein Verfahren zur quantitativen Bestimmung von menschlichen oder tierischen Antikörpern in Verbindung mit einer an sich bekannten immunologischen Bestimmungsmethode, wie einem Elisa-Test. Zur quantitativen Durchführung der Bestimmungsmethode ist bei dem Kit ein Antigen-unspezifischer Antikörper in vorbestimmten Mengen an feste Träger gebunden, die in trockener haltbarer Form vorliegen. Die Antigen-unspezifischen Antikörper reagieren in gleicher Weise mit einem Enzym-markierten Anti-Antikörper wie der zu bestimmende Antikörper, so daß dessen Menge durch direkten Farbvergleich quantitativ bestimmbar ist.

EP 0 341 470 A1

EP 0 341 470 A1

**Kit und Verfahren zur quantitativen Bestimmung von Antikörpern**

Die Erfindung betrifft einen Kalibrierungs-Kit zur quantitativen Bestimmung von Antikörpern u.dgl. in menschlichen und tierischen Seren oder anderen Flüssigkeiten in Verbindung mit einer immunologischen Bestimmungsmethode, insbesondere eines heterogenen Elisa-Tests, bei der ein Träger, der mit einem bestimmten Antigen beladen ist, mit dem der zu bestimmende Antigen-spezifische Antikörper immunologisch zu reagieren vermag, zur Bestimmung von etwa gebundenem Antikörper mit einem Anti-Antikörper versetzt wird, sowie ein Verfahren zur Durchführung der Bestimmung.

Die Erfindung betrifft insbesondere einen Kalibrierungs-Kit, der zusammen mit einem Kit für einen indirekten heterogenen Elisa-Test anwendbar ist.

Immunologische Bestimmungsmethoden zur qualitativen bzw. halbquantitativen Bestimmung von Antikörpern, beispielsweise durch indirekte heterogene Elisa, sind seit längerer Zeit bekannt und eingehend beschrieben. Für die Verlaufskontrolle einer Infektionserkrankung oder einer Allergie reichen die derzeitigen Ja- Nein-Aussagen oder semiquantitativen Bestimmungen im Titer nicht aus, da die IgM-, IgA-, IgG- und IgE-Antikörper für Monate und Jahre gebildet werden. Um die Konzentration der Antigen-spezifischen Antikörper über eine längere Zeit quantitativ erfassen zu können, ist der exakte Nachweis der spezifischen Antikörper in SI-Einheiten oder in internationale Einheiten (IU/ml) erforderlich.

Bisher wurde die Ja-/Nein-Aussage mit Hilfe eines Cut off's ermittelt, z.B. mit Hilfe der optischen Dichte im Fotometer. Damit kann keine Aussage über Konzentration spezifischer Antikörper getroffen werden. Eine weitere diagnostische Auswertung erfolgt als semiquantitative Titerbestimmung. Hierbei werden mit Hilfe eines feststehenden Cut off's und Verdünnungsreihen Titer ermittelt. Bei dieser Auswertungsmethode kann jedoch die Fehlerquote ± eine Titerstufe also ± 50 % betragen.

Bei der Auswertung mit Hilfe von Standards, wozu wenigstens drei Standards notwendig sind, wird eine Eichkurve erstellt, an der Konzentrationen abgelesen werden können. Dies trifft zum jetzigen Zeitpunkt der Diagnostik nur für den Bereich Total IgE-Antikörper, Tumormarker und Hormone zu, nicht jedoch für den Bereich der Infektionskrankheiten und allergenspezifischer IgE-Antikörper, von wenigen Ausnahmen abgesehen. Auch bei dieser Methode müssen erhebliche Einschränkungen in Kauf genommen werden. So ist das Auffinden von hochtitrigen Antigen-spezifischen Antikörpern vor allem für IgM, IgA und IgE stark vom Zufall abhängig. Weiterhin ist die Stabilität des jeweils angebotenen Standards als biologisches Material deutlich in der Haltbarkeit begrenzt wird kann bei Abbau bzw. geringer Haltbarkeit der Standard-Sera zu falschen Ergebnissen führen, und zwar sowohl nach oben als auch nach unten.

Der Erfindung liegt deshalb die Aufgabe zugrunde, eine allgemein brauchbare quantitative Bestimmungsmethode zur Bestimmung von Antikörpern zu schaffen, insbesondere für die Diagnose von Infektionskrankheiten und zur Bestimmung von allergenspezifischen Antikörpern. Weiterhin soll die Bestimmungsmethode ohne großen zeitlichen und finanziellen Aufwand durchführbar sein.

Die Erfindung stellt einen Kalibrierungs-Kit und ein Verfahren zur quantitativen Bestimmung von menschlichen oder tierischen Antikörpern o.dgl. im Serum oder anderen Flüssigkeiten, insbesondere Körperflüssigkeiten, in Verbindung mit einer bekannten immunologischen Bestimmungsmethode, insbesondere einem heterogenen Elisa-Test, zur Verfügung.

Der Kit ist dadurch gekennzeichnet, daß Antigen-unspezifische Human- bzw. Tier-Antikörper in bekannten Mengen insbesondere in aufsteigender Reihe an feste Träger gebunden sind und die jeweils beladenen Träger in trockener, haltbarer Form vorliegen. Der zur immunologischen Reaktion mit dem zu bestimmenden Antikörper fähige markierte Anti-Antikörper (Konjugat) ist auch zur Reaktion mit dem unspezifischen Human- bzw. Tier-Antikörper vorgesehen, so daß die Menge des zu bestimmenden Antikörpers durch direkten Vergleich des gefundenen Wertes mit dem entsprechenden bekannten Wert des vorgelegten unspezifischen Antikörpers bestimmbar ist. Als Antigene bzw. anstelle dieser können auch Tumorantigene oder Allergene an den Träger gebunden sein. Der Kalibrierungs-Kit ist ein Kit für die Durchführung eines heterogenen direkten Elisa-Tests und ist eine Ergänzung für verschiedenartige bekannte immunologische Bestimmungsmethoden, um deren Ergebnisse quantitativ auswerten zu können.

Das Verfahren zur Durchführung der Bestimmung besteht darin, daß mindestens ein mit dem Antigen belegter Testträger mit einer auf den zu bestimmenden Antikörper zu untersuchenden Flüssigkeit versehen wird, nach einer ggf. erfolgten immunologischen Bindung des Antikörpers sowohl dieser Träger als auch die für die Durchführung einer Kalibrierung verwendeten vorgefertigten Träger, die mit Antigen-unspezifischem Antikörper in aufsteigender Menge beladen sind, im wesentlichen gleichzeitig mit einem markierten spezifischen Anti-Antikörper behandelt werden und die Menge an gesuchtem Antikörper durch direkten Vergleich der Farbintensität beim Testträger mit den verschiedenen Intensitäten der für die Kalibrierung verwendeten Träger mit den bekannten Mengen an Antikörper bestimmt wird. Gleichzeitig mit dieser

2

EP 0 341 470 A1

Bestimmung können in an sich bekannter Weise ein Anti-Antikörper-Leerwert und eine Positiv- und eine Negativ-Kontrolle durchgeführt werden. Bei den Antigen-unspezifischen Antikörpern handelt es sich um solche, die denselben Lebewesen zuzuordnen sind, wie der gesuchte Antikörper. Werden menschliche Antikörper gesucht, dann ist es ein Humanantikörper. Bei tierischen Antikörpern ist der Antigen-unspezifische Antikörper derselben Tierart, insbesondere Rasse, zugeordnet wie der gesuchte Antikörper. Dies gilt auch für den Enzym-markierten Anti-Antikörper. Der Kalibrierungs-Kit und Test ist somit unabhängig vom jeweiligen Antigen und dem gesuchten Antigen-spezifischen Antikörpers innerhalb des Human- bzw. jeweiligen Tierbereiches.

Es wurde insbesondere gefunden, daß humane oder tierische, nicht Antigen-spezifische, also unspezifische, Antikörper, insbesondere von den IgM-, IgA-, IgG- und IgE-Klassen, wenn sie auf eine feste Phase kodiert sind, sich zur Durchführung von Kalibrierungstests eignen. Dabei bewegt sich die Konzentration dieser Antikörper vorzugsweise in den Bereichen, wie sie bei der Bindung anti-Antigen-spezifischer Antikörper im Elisa-/Ria-/Lumineszenz-Test zu beobachten sind. Die Reak tion mit dem Indikator-Antikörper (Anti-Antikörper; Anti-Humankonjugat) verläuft vorzugsweise über einen weiten Bereich linear. Hierzu sind erfindungsgemäß die unspezifischen Antikörper vorzugsweise in verschiedenen feststehenden Konzentrationen an die jeweiligen festen Phasen kodiert. Als Träger für das Antigen und für den unspezifischen Antikörper sind jeweils vorzugsweise die gleichen Träger vorgesehen, insbesondere Mikrotiterplatten bzw. -streifen. Perlen (Beads), Röhrchen, Teststäbchen, Folien, Papiere, Becher, Sticks und dergleichen. Diese Träger können aus den bekannten Materialien bestehen. Die Bindung des Antigens bzw. des unspezifischen Antikörpers an den Träger kann physikalischer Art (isoelektrisch/ adsorptiv) oder eine kovalente chemische Bindung sein.

Da die unspezifischen Antikörper und/oder das Antigen in trockener Form in vorbestimmten Mengen an den Träger gebunden sind, sind sie außerordentlich lange haltbar. Dadurch, daß die Träger mit den gebundenen Antikörpern bzw. Antigenen beim jeweiligen Kit bereits vorgefertigt vorliegen und mit diesem bezogen werden, entfällt eine Zubereitung von Standards, wie dies zur Herstellung einer Eichkurve bisher erforderlich war. Auch brauchen keine Antikörper-Lösungen mehr für solche Standards zubereitet werden, die schnell unbrauchbar werden bzw. hre Werte verändern Weiterhin ist der erfindungsgemäße Kit unabhängig von den bisher verwendeten spezifischen Antikörpern, die je nach ihrer Genese unterschiedliche Aktivitäten aufweisen. Vielmehr können in großer Menge gewonnene unspezifische Antikörper gleichbleibender Aktivität eingesetzt werden, wodurch feststehende und reproduzierbare Standard-Kurven mit quantitativen Aussagen bei der Bestimmung von Antikörpern, insbesondere in der Infektionsserologie und Allergiediagnostik, getroffen werden können. Für die Tumordiagnostik zum Beispiel und deren Ver laufskontrolle können anstelle der Ig-Humanantikörper die jeweiligen Tumorantigene in genauen Konzentrationen als Standards an die feste Phase kodiert werden.

Die Erfindung bietet somit eine gleichbleibende Stabilität, insbesondere der verwendeten Reagentien und Verfahrensbedingungen, eine Unabhängigkeit vom Auffinden humaner positiver Kontrollen für Standards, was insbesondere für IgM, IgA und IgE zutrifft. Die Erfindung ermöglicht außerdem eine Reproduzierbarkeit der Standard-Kurven und der Konzentrationen für die Verlaufskontrollen. Weiterhin wird auch eine Vergleichbarkeit der bekannten Tests in der Verlaufskontrolle ermöglicht. Diese Vorteile werden geboten, ohne daß der erfindungsgemäße Kit bzw. die Bestimmung mit Mehrkosten verbunden ist.

Die Erfindung ist weiterhin als universelle Methode für alle Antigene und Allergene, für alle markierten Antikörper bei den Testmethoden Elisa, Ria und Lumineszenz anwendbar. Durch die bereits vorher vorgenommene Bindung der Antikörper sowie Tumorantigene bzw. Allergene an feste Phasen verschiedener Art ist die Methode außerdem für alle Testsysteme geeignet.

Die Menge des Antigens bzw. Allergens, die an den festen Träger gebunden ist, liegt in einem für das jeweilige Antigen bzw. Allergen günstigen bzw. optimalen Bereich für den Test. Dieser Bereich kann leicht in an sich bekannter Weise durch Vorversuche bestimmt werden. Die aufsteigende Reihe der Mengen des unspezifischen Antikörpers ist vorzugsweise eine geometrische Reihe. Die Reihe entspricht insbesondere dem linearen Teil einer sigmoiden Kurve.

In der vorgefertigten trockenen Form der mit dem Antigen (Allergen) bzw. dem unspezifischen Antikörper beladenen Träger sind etwaige freie Bindungsstellen der Träger vorzugsweise bereits markiert, um ungewünschte Bindungen während der Bestimmung zu vermeiden. Als trockene Form ist der lyophilisierte Zustand bzw. die gefriergetrocknete Form und Vakuum-Verpackung besonders bevorzugt. Im übrigen kann die Bestimmungsmethode in an sich bekannter Weise durchgeführt werden.

Da die Antikörper unspezifischer Natur sind, können die gleichen mit der aufsteigenden Reihe der Antikörper beladenen Träger für die Bestimmung verschiedener Antikörper eingesetzt werden, d.h. im Zusammenhang mit Trägern, die mit verschiedenen Antigenen für die Durchführung verschiedener Bestimmungen beladen sind. Für den jeweiligen Test spezifische Antikörper sind für die Kalibrierung nicht erforderlich. Für die Standard-Kurve werden vorzugsweise mindestens vier, insbesondere fünf bis zehn verschiedene bekannte Mengen an unspezifischen Antikörper vorgelegt. Werden Mikrotiterplatten bzw. -streifen verwendet, dann eignen sich sieben vorgelegte bekannte Mengen an unspezifischem Antikörper und ein Null-Wert, so daß eine Reihe von A bis H belegt ist. Die unspezifischen Antikörper können auch in die einzelnen Subklassen, insbesondere die Subklassen IgG 1 bis 4 aufgetrennt sein.

Weitere Merkmale der Erfindung ergeben sich aus der nachfolgenden Beschreibung von bevorzugten speziellen Ausführungen anhand eines heterogenen indirekten Elisa-Tests in Verbindung mit den Ansprüchen. Als Beispiel wird der Human-Antikörper gegen Eppstein-Barr-Virus-Antigen bestimmt.

Für die Durchführung eines indirekten heterogenen Elisa-Tests werden die Vertiefungen von zur Kalibrierung verwendeten Mikrotiterstreifen (z.B. aus Polystyrol) mit verschiedenen, bekannten Konzentrationen von nicht-spezifisch reagierenden Immunglobulin-Klassen adsorptiv beschichtet. Dabei wird der Bereich der Antikörperkonzentration so gewählt, daß er die Konzentrationen der im indirekten heterogenen Elisa an das Antigen gebundenen Immunglobuline erfaßt. Auf diese Weise ist eine exakt quantitative Aussage über spezifische Antikörper in µg/ml, µmol oder auch IU/ml möglich.

Zur Herstellung der Kalibrierungsstreifen werden zunächst in an sich bekannter Weise IgG- und IgM-Antikörper aus Humanserum isoliert und durch DEAE-Ionenaustauschchromatographie getrennt. Die Anreicherung der Immunglobulinfraktionen erfolgt beispielsweise durch Ausfällen mit gesättigter Ammoniumsulfat-Lösung bei 4 °C.

Nach Aufnahme in physiologischer NaCl-Lösung werden die einzelnen Fraktionen über Sephadex G 25-Säule entsalzt. Die Proteinbestimmung der Immunglobulin-Klassen erfolgt mit der Biuret-Methode gegen Rinderserumalbumin als Standard. Für die Beschichtung der Mikrotiterstreifen wird der Proteingehalt eingestellt (ca. 1200 ng/ml).

Mit Hilfe einer geometrischen Verdünnungsreihe mit 0,05 M Bicarbonatpuffer pH 9,6 werden aus der Immunglobulin-Ausgangslösung folgende Konzentrationen für die Beschichtung einer aus zwölf Mikrotiterstreifen zusammengesetzten Mikrotiterplatte hergestellt.

| Reihe | Konzentration ng/ml |
|---|---|
| A 1 - 12 | 0 |
| B 1 - 12 | 600 |
| C 1 - 12 | 300 |
| D 1 - 12 | 150 |
| E 1 - 12 | 75 |
| F 1 - 12 | 37,5 |
| G 1 - 12 | 18,75 |
| H 1 - 12 | 9,38. |

Diese Verdünnungen werden in acht verschiedenen Vorratsgefäßen an einen 8-Kanal-Dispenser angeschlossen.

Die jeweiligen Immunglobulin-Konzentrationen werden in Volumina von 100 µl in die entsprechenden Vertiefungsreihen der Mikrotiterplatte dispensiert.

Die Beschichtung erfolgt durch eine nicht-kovalente Adsorption nahe dem pI-Wert der Proteine.

Für die Beschichtung werden 60 CO-bestrahlte Flachboden-Mikrotiterplatten mit einer maximalen Bindungskapazität von 800 ng Protein pro Vertiefung verwendet. Die Inkubationszeit beträgt 16 - 20 Stunden bei Raumtemperatur.

Danach werden die Mikrotiterplatten drei Mal mit 300 µl Waschlösung pro Vertiefung gewaschen, auf einer Zellstoffunterlage kurz trocken geklopft und mit 250 µl pro Vertiefung einer 2 %igen Caseinlösung mit 3 % Karion F (Sorbitsirup) nachbeschichtet, um die noch freien Zwischenräume zwischen den adsorbierten Immunglobulinen zu besetzen. Die Nachbeschichtung erfolgt durch physikalische Adsorption nahe dem pI-Wert des Caseins bei 37 °C über eine Zeit von 2 Stunden.

Dann wird die Nachbeschichtungslösung aus der Mikrotiterplatte abgesaugt, die Platten auf einer Zellstoffunterlage kurz trocken geklopft und über 3 Stunden in einem Lyophilisator lyophil getrocknet. Anschließend werden die Platten mit Trockenmittel in Sauerstoff undurchlässigen Siegelrand-Beuteln aus Polyethylen vakuumverpackt.

Der Test kann in an sich bekannter Weise durchgeführt werden. Vor dem zweiten Reaktionsschritt beim indirekten heterogenen Elisa (vor der Zugabe von enzymmarkiertem antihuman IgG-/IgM-Antikörpern) werden die Kalibrierungsstreifen für die IgG- und IgM-Bestimmung in die Streifenhalterung eingesetzt und wie die anderen Elisa-Streifen (Antigen-beschichtet) mit 100 µl Konjugatverdünnung 30 Minuten bei 37 °C inkubiert.

Es wird vier Mal mit 350 µl Waschlösung gewaschen. Dann werden 100 µl Substratlösung zugegeben. 15 Minuten wird bei 37 °C inkubiert.

Reaktion wird mit 50 µl 1 M $H_2SO_4$ gestoppt.

Mit dem Elisa-Reader werden alle Streifen bei 492 nm gemessen. (Die Wellenlänge richtet sich nach dem Enzymsubstrat.)

Alle OD-Werte für IgG werden durch 2,0 dividiert und mit 100 multipliziert (B/Bo x 100).

Alle OD-Werte für IgM werden durch 1,5 dividiert und mit 100 multipliziert (B/Bo x 100),

| B | = | gemessener Wert |
|---|---|---|
| Bo | = | 2,0 für IgG |
| | | 1,5 für IgM. |

Eine Standard-Kurve B/Bo x 100 gegen Konzentration ng/ml wird auf halblogarithmischem Papier erstellt. Die Werte der Testsera aus dem indirekten Elisa werden aus der Standard-Kurve abgelesen und mit dem Verdünnungsfaktor (von 100) multipliziert. Die Konzentration der spezifischen Antikörper ist µg/ml

Auf den Kalibrierungsstreifen sind folgende IgG- und IgM-Konzentrationen aufgetragen:

| A | 0 | ng/ml |
|---|---|---|
| B | 600 | ng/ml |
| C | 300 | ng/ml |
| D | 150 | ng/ml |
| E | 75 | ng/ml |
| F | 37,5 | ng/ml |
| G | 18,75 | ng/ml |
| H | 9,38 | ng/ml. |

Durch Farbvergleich kann der ermittelte Wert aus der entsprechenden Kurve direkt abgelesen werden.

Durchführungen dieses Tests zur Bestimmung von Antikörpern gegen Eppstein-Barr-Virus-Antigen bestätigte die gute Reproduzierbarkeit und die Richtigkeit der gefundenen Werte. In entsprechender Weise können andere Antikörper bestimmt werden.

| 1. Reagentien | |
|---|---|
| 0,05 M Bicarbonatpuffer pH 9,6 : | |
| $Na_2CO_3$ | 1,6 g |
| $NaHCO_3$ | 2,9 g |
| Aqua dest. auf | 1 l |
| 1 Spur Phenolrot zugeben, mischen. | |

| 2. Nachbeschichtungslösung | | |
|---|---|---|
| PBS-Puffer | pH 7,2 | (10 x konz.) |
| (Phosphat-gepufferte Kochsalzlösung) | | |
| Na₂HPO₄ x 2H₂ | 82,2 g | |
| KH₂PO₄ | 12,0 g | |
| Aqua dest. auf | 1 l. | |
| PBS-Puffer 1:10 mit Aqua dest. verdünnen. 20 g Casein lösen und 30 ml Karion F in 1l Puffer lösen. pH-Wert 7,2 - 7,4. | | |

| 3. Waschlösung (20 x konz.) | | |
|---|---|---|
| NaCl | 150 | g |
| KH₂PO₄ | 4 | g |
| Na₂HPO₄ x 2H₂O | 23 | g |
| Thimerosal | 0,1 | g |
| 100 mM PMSF in Methanol | 0.4 | ml |
| Tween 20 | 20 | ml |
| Aqua dest. auf | 1 | l. |
| Vor Gebrauch Lösung 1:20 verdünnen. Der pH-Wert der Gebrauchslösung soll bei 7,2 liegen. | | |

**Ansprüche**

1. Kit zur quantitativen Bestimmung von menschlichen oder tierischen Antikörpern u.dgl. in Seren oder anderen biologischen Flüssigkeiten in Verbindung mit einer an sich bekannten immunologischen Bestimmungsmethode, insbesondere einem heterogenen Elisa-Test, bei der ein Träger mit einem bestimmten Antigen beladen ist, mit dem der zu bestimmende Antigen-spezifische Antikörper immunologisch zu reagieren vermag, und für die immunologische Reaktion mit dem dadurch gebundenen Antigen-spezifischen Antikörper ein Enzym-markierter Anti-Antikörper (Konjugat) vorgesehen ist, dadurch gekennzeichnet, daß Antigen-unspezifische Antikörper humaner bzw. tierischer Art an feste Träger in vorbestimmten Mengen gebunden sind, die jeweiligen beladenen Träger in trockener haltbarer Form vorliegen und für die gleiche Inkubationsphase mit dem zu bestimmenden Antigen-spezifischen Antikörper zur immunologischen Reaktion mit dem Enzym-markierten Anti-Antikörper (Konjugat) vorgesehen sind, so daß die Menge des zu bestimmenden Antikörpers durch direkten Vergleich des gefundenen Wertes mit dem entsprechenden Wert des vorgelegten unspezifischen Antikörpers bestimmbar ist.

2. Kit nach Anspruch 1, dadurch gekennzeichnet, daß der Träger für das Antigen und für den unspezifischen Antikörper derselben Art ist, insbesondere eine Mikrotiterplatte, -streifen oder Kugeln.

3. Kit nach Anspruch 1 oder 2, dadurch gekennzeichnet, daß das Antigen und/oder der Antigen-unspezifische Antikörper physikalisch oder kovalent an den Träger gebunden sind.

4. Kit nach einem der vorhergehenden Ansprüche, dadurch gekennzeichnet, daß der unspezifische Antikörper und/oder das Antigen in lyophilisierter Form an den Träger gebunden vorliegen.

5. Kit nach einem der vorhergehenden Ansprüche, dadurch gekennzeichnet, daß die Konzentrationen des vorgelegten unspezifischen Antikörpers in einer aufsteigenden Reihe, insbesondere einer geometrischen Reihe, vorliegen.

6. Kit nach einem der vorhergehenden Ansprüche, dadurch gekennzeichnet, daß mindestens vier, vorzugsweise fünf bis zehn verschiedene bekannte Mengen an unspezifischem Antikörper vorgelegt sind.

7. Kit nach einem der vorhergehenden Ansprüche, dadurch gekennzeichnet, daß sowohl beim in trockener Form vorliegenden Träger für das Antigen als auch bei den Trägern für den Antikörper freie Bindungsstellen am Trägermaterial maskiert sind.

8. Kit nach einem der vorhergehenden Ansprüche, dadurch gekennzeichnet, daß der unspezifische Antikörper aus der Reihe IgG, IgM, IgA, IgE bzw. einer Subklasse davon ist.

9. Verfahren zur Durchführung der Bestimmung von humanen bzw. tierischen Antikörpern, insbesondere in Serum, bei dem mindestens ein mit einem Antigen belegter Testträger mit einer auf den zu bestimmenden Antikörper zu untersuchenden Flüssigkeit versehen wird und nach einer ggf. erfolgten immunologischen Bindung des Antikörpers der gebundene Antikörper mit Hilfe eines markierten human- bzw. tierspezifischen Anti-Antikörpers bestimmt wird, dadurch gekennzeichnet, daß für die Durchführung einer Kalibrierung verwendete vorgefertigte Träger, die mit unspezifischem Human- bzw. Tier-Antikörper in aufsteigender Menge beladen sind, im wesentlichen gleichzeitig wie der gebundene Antikörper mit dem markierten human- bzw. tierspezifischen Anti-Antikörper behandelt werden und die Menge an gesuchtem Antikörper durch direkten Vergleich der Farbintensität beim Testträger mit den verschiedenen Intensitäten der für die Kalibrierung verwendeten Träger mit bekannten Mengen an Antikörper bestimmt wird.

10. Verfahren nach Anspruch 9, dadurch gekennzeichnet, daß bei der Bestimmung des Antikörpers außer dem Kalibrierungstest mindestens ein Anti-Antikörper-Leerwert be stimmt als auch eine negative Kontrolle und eine positive Kontrolle in an sich bekannter Weise durchgeführt werden.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | JOURNAL OF IMMUNOLOGICAL METHODS, Band 82, 1985, Seiten 233-241, Elsevier Science Publishers B.V. (Biomedical Division), Amsterdam, NL; M. REZA ZIAI et al.: "An enzyme-linked double antibody immunoassay to measure murine immunoglobulins - Its application to determine the specific activity of radiolabeled monoclonal antibodies" * Seite 236, Zeilen 16-36; Figuren 2-4 * | 1-3,5-10 | G 01 N 33/58<br>G 01 N 33/68<br>G 01 N 33/531<br>G 01 N 33/96 |
| | --- | | |
| X | CHEMICAL ABSTRACTS, Band 108, 1988., Seite 424, Zusammenfassung Nr. 4347q, Columbus, Ohio, US; A. FOMSGAARD et al.: "ELISA for human IgG and IgM anti-lipopolysaccharide antibodies with indirect standardization", & J. IMMUNOASSAY 1987, 8(4), 333-50 * Zusammenfassung * | 1-3,5-10 | |
| | --- | | RECHERCHIERTE SACHGEBIETE (Int. Cl.4) |
| Y | JOURNAL OF IMMUNOLOGICAL METHODS, Band 93, 1986, Seiten 207-212, Elsevier Science Publishers B.V. (Biomedical Division), Amsterdam, NL; A. FERRANTE et al.: "An enzyme-linked immunosorbent assay for the quantitation of human IgG subclasses using monoclonal antibodies" * Seite 208, Spalte 1, Zeile 28 - Spalte 2, Zeile 33; Figuren 1-4 * --- -/- | 1-10 | G 01 N |

Der vorliegende Recherchenbericht wurde für alle Patentansprüche erstellt

| Recherchenort | Abschlußdatum der Recherche | Prüfer |
|---|---|---|
| DEN HAAG | 28-08-1989 | VAN BOHEMEN C.G. |

**Europäisches Patentamt**

Nummer der Anmeldung

EP 89 10 7298

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | JOURNAL OF IMMUNOLOGICAL METHODS, Band 90, 1986, Seiten 71-76, Elsevier Science Publishers B.V. (Biomedical Division), Amsterdam, NL; J. SANCHO et al.: "Quantitative measurement of human immunoglobulin E using monoclonal antibodies to distinct epitopes" * Seite 72, Spalte 2, Zeilen 18-41; Figuren 1-5 * ----- | 1-10 | |
| | | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| DEN HAAG | 28-08-1989 | VAN BOHEMEN C.G. |